# DEMANDE DE BREVET EUROPEEN

(11) **EP 2 722 328 A1**
(43) Date de publication de la demande: **23.04.2014**
(21) Numéro de dépôt: 12306297.8
(22) Date de dépôt: 19.10.2012
(51) Int. Cl.: C07D 239/91, C07D 409/04, C07D 409/14, C07D 417/14, A61K 31/427, A61K 31/517, A61K 31/381, A61K 31/4439, A61K 31/506, A61P 39/02

(54) **Nouveaux composés ayant une activité protectrice vis-à-vis de toxines au mode d'action intracellulaire**

(71) Demandeur: COMMISSARIAT A L'ENERGIE ATOMIQUE ET AUX ENERGIES ALTERNATIVES, 75015 Paris (FR); Institut Curie, 75248 Paris Cedex 05 (FR)
(72) Inventeur: Gillet, Daniel, 75003 PARIS (FR); Barbier, Julien, 91190 GIF-SUR-YVETTE (FR); Johannes, Ludger, F-92400 COURBEVOIE (FR); Cintrat, Jean-Christophe, 91430 IGNY (FR); Noel, Romain, 22660 TREVOU-TREGUINEC (FR)
(74) Mandataire: Bernstein, Claire Jacqueline

(57) **Abrégé**

La présente invention a pour objet une nouvelle famille de composés du type 2,3-dihydroquinazolin-4(1*H*)-one de formule générale (I) 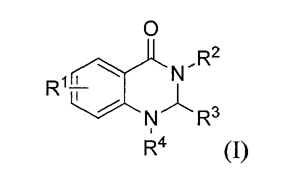 et leur utilisation en tant qu'inhibiteurs des effets toxiques des toxines à activité intracellulaire, comme par exemple la ricine, utilisant le transport rétrograde pour intoxiquer les cellules.

## Description

La présente invention a pour objet une nouvelle famille de composés du type 2,3-dihydroquinazolin-4(1*H*)-one et leur utilisation en tant qu'inhibiteurs des effets toxiques des toxines à activité intracellulaire, comme par exemple la ricine, utilisant le transport rétrograde pour intoxiquer les cellules.

Les toxines à activité intracellulaire qui utilisent le transport rétrograde représentent un problème majeur de santé publique et sont associées à plus d'un million de décès chaque année dans le monde. Ces toxines sont notamment la ricine (produite dans les graines de la plante *Ricinus communis*)*,* la toxine de Shiga et les toxines Shiga-like (Stxs) produite par *Shigella dysenteriae* (Stx) et *E. coli* (Stx1 et Stx2), la toxine cholérique (Ctx de *Vibrio cholerae* responsable du choléra), la toxine pertussique (*Bordetella pertussis* agent de la coqueluche), la cytotoxine subtilase et l'entérotoxine thermolabile *(E. coli*)*.*

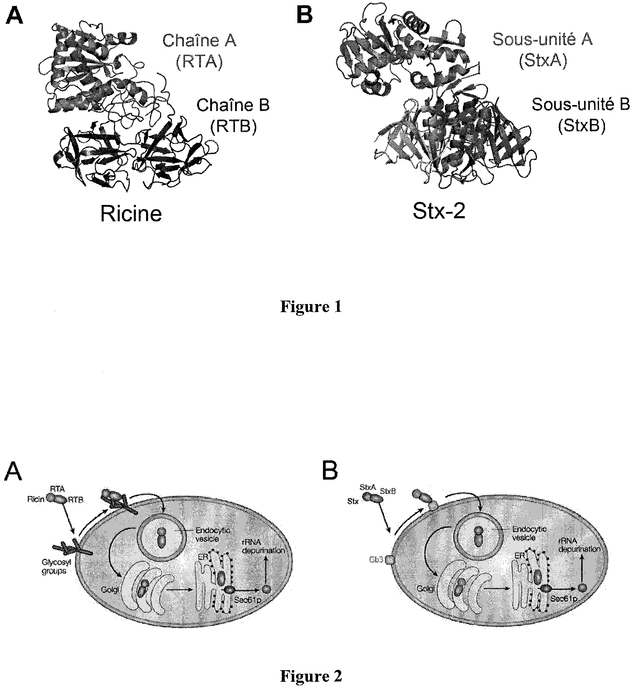

La ricine est une glycoprotéine de 66 kDa composée de deux chaînes polypeptidiques reliées par un pont disulfure **(****Figure 1A****).** La chaîne B (RTB, lectine de 262 résidus) permet à la toxine de se lier aux glycolipides et glycoprotéines des membranes cellulaires et d'assurer son entrée dans la cellule **(****Figure 1A****).** La chaîne A (RTA, 267 acides aminés) assure la fonction enzymatique N-glycosidase de la ricine, catalyse l'élimination de l'adénine 4324 de l'ARN 28S des cellules ciblées et provoque l'arrêt de la synthèse des protéines.

Les toxines de Shiga incluent la toxine de Shiga (Stx) produite par *Shigella dysenteriae* et les toxines Shiga-like 1 (Stx1) et 2 (Stx2) produites par les souches entérohémorragiques d'*E*. *coli.* Les toxines Stx et Stx1 sont à 99% identiques alors que les Stx1 et Stx2 partagent seulement 56% d'identité de leur séquence en acides aminés. La sous-unité A de la toxine (StxA) porte la même activité enzymatique que la ricine et cible de manière identique l'ARN 28S **(****Figure 1B****).** La sous-unité B (StxB), qui se présente sous forme pentamérique, permet la liaison de la toxine avec la cellule *via* son interaction avec le globo-triaosylcéramide Gb3, ce qui assure son intemalisation et son routage intracellulaire.

Après liaison à ses récepteurs membranaires, la ricine est intemalisée dans ces cellules par de multiples voies d'endocytose pour atteindre le réseau *trans-*golgien où elle est acheminée vers le réticulum endoplasmique (RE) par transport rétrograde **(****Figure 2A**). Les toxines de Shiga sont internalisées quant à elles par une voie d'endocytose unique et atteignent également l'appareil de Golgi puis le réticulum endoplasmique **(****Figure 2B****).**

Les toxines sont alors partiellement dépliées et la chaîne/sous-unité A est transloquée dans le cytosol (Lord, J.M. et al., Biochemical Society Transactions 2003, 31, 1260). L'étape ultime de l'action de ces toxines a donc lieu dans le cytoplasme des cellules, les toxines se fixent sur les ribosomes avec une grande efficacité et clivent l'adénine 4324 de l'ARN 28S de la sous-unité 60S du ribosome. Cette dépurination de l'ARN 28S provoque l'arrêt de la synthèse des protéines et conduit à la mort cellulaire.

Pour contrer la menace posée par ces toxines, plusieurs types d'antitoxines ont été développés : anticorps neutralisants, inhibiteurs de l'activité enzymatique (petites molécules, analogues de substrat), mimes solubles de récepteurs et composés chimiques agissant sur les cellules ciblées par la toxine :
● Petites molécules inhibitrices de l'activité enzymatique de la ricine (Miller, D. et al., J. Med. Chem 2002, 45, 90 et Yan, X. et al., J. Mol. Biol. 1997, 266, 1043).
● Petites molécules inhibitrices de l'activité enzymatique de Stx (Brigotti M. et al., Nucleic Acids Res. 2000, 28 (12), 2383).
● Dérivés d'ARN : Comme la ricine reconnaît une séquence bien précise au niveau de l'ARN28S, la boucle SRL *(Sarcin-Ricin Loop),* des inhibiteurs ont été conçus en se basant sur cette séquence nucléotidique (Schramm et al. Biochemistry 2001, 40 (23), 6845;Roday, S. et al., Biochemistry 2004, 43, 4923*;* Hesselberth, J. R. et al., J. Biol. Chem. 2000, 275, 4937, Fan, S. et al., World J. Gastroenterol. 2008, 14, 6360).
● Anticorps dirigés contre la ricine (Lemley, P.V. et al., Hybridoma 1994, 13, 417 ; Guo, J.W. et al., Hybridoma 2005, 24, 263 ;Pelat, T. et al., BMC Biotechnol. 2009, 9, 60).
● Anticorps dirigés contre Stx (Sheoran A.S. et al., Infect Immun. 2005, 73, 4607-13 ; Tzipori S. et al., Clin. Microbiol. Rev. 2004 17, 926-41)*.*
● Mimes solubles de récepteurs de la ricine (Perera L.P. et al., J Clin Microbiol. 1988, 26, 2127-31; Smith M.J. et al., Infect Immun. 2006, 74, 6992-8).
● Mimes solubles de récepteurs de Stx (Armstrong G.D. et al., J. Infect. Dis. 1995, 171, 1042-5 ; Trachtman H. et al., JAMA 2003, 290, 1337-44 ; Kitov P.I. et al., Nature 2000, 403, 669-72 *;* Nishikawa K. et al., PNAS 2002, 99, 7669-74 ; Mulvey G.L. et al., J. Infect. Dis. 2003, 187, 640-9 ; Watanabe et al., J. Infect. Dis. 2004, 189, 360-8).
● Approche vaccinale pour contrer les effets de la ricine : un certain nombre de vaccins ont été décrits notamment dans des brevets et des publications scientifiques. Ils revendiquent une protection vis-à-vis de la ricine *via* l'administration d'une quantité immunogénique de dérivés de RTA ou RTB.
● Approche vaccinale pour contrer les effets de Stx : un vaccin efficace chez l'homme devrait conduire à la production d'anticorps prévenant la colonisation des bactéries dans le tractus intestinal ou neutralisant les toxines de Shiga produites, afin d'empêcher le développement du syndrome hémolytique et urémique. Un tel vaccin n'est pas disponible à ce jour même si des travaux de recherche sont actuellement menés dans cette direction.

Ces dernières années, la recherche de nouvelles molécules visant à bloquer le routage intracellulaire des toxines à activité intracellulaire s'est accélérée. Le principal avantage de ce type de molécules est leur activité de type large spectre puisque ces molécules peuvent protéger efficacement les cellules contre les différentes toxines qui utilisent la voie rétrograde.

Cependant ces molécules, telles que la Bréfeldine A (Donta S.T. et al., J. Infect. Dis.1995 171, 721-4), Exo2 (Spooner R.A. et al., Biochem J. 2008, 414, 471-84 ;. Yarrow J.C. et al., C.C.H.T.S. 2003, 6, 279-86), Golgicide (Saenz J.B. et al., Nat. Chem. Biol. 2009, 5, 157-65) et autres (Saenz J.B. et al., Infect. Imm. 2007, 75, 4552-61), même si elles protègent les cellules contre l'action des toxines à activité intracellulaire, sont elles-mêmes cytotoxiques car elles ciblent des fonctions clé de l'homéostasie cellulaire et ne peuvent donc pas être utilisées dans un but thérapeutique.

D'autres molécules ont été identifiées notamment par criblage à haut débit en tant qu'inhibiteur de l'action de la ricine et/ou des Stx : les composés 134 et 75 (Saenz J.B. et al., Infect. Imm. 2007, 75, 4552-61) *(voir le schéma ci-après*)*.* La cible cellulaire de ces molécules demeure inconnue ; elles semblent bloquer le transport de la ricine et des Stx à l'intérieur des cellules. Cependant, ces composés ne protègent pas les cellules épithéliales pulmonaires humaines A549 et aucune de ces molécules n'a démontré un effet protecteur chez l'animal. De plus, ces molécules modifient la morphologie de l'appareil de Golgi ce qui indique, d'une part, un mécanisme d'action différent et, d'autre part, une toxicité intrinsèque des composés.

Le criblage haut débit a également permis d'identifier les composés Retro-1 et Retro-2 (EP2145873, WO2009/153457, WO2009/153665, Stechmann B. et al., Cell 2010, 141, 231-42) en tant qu'inhibiteurs de la ricine et/ou de Stx ; ces composés sont capables de protéger les cellules épithéliales pulmonaires humaines A549 ; en outre, Retro-2 a montré une efficacité chez l'animal.

Une autre équipe de recherche a identifié de nouvelles molécules protectrices vis-à-vis de la ricine et des Stxs également lors d'un criblage à haut débit sur cellules Vero (Wahome P.G. et al., Toxicon 2010, 56, 313-23). Ces composés (CID 644401, CID 5737931 et CID 18576762) ne semblent pas aussi actifs (EC50 compris entre 25 et 60 µM) que les composés Rétro-1 et Retro-2 et leur mode d'action reste inconnu.

### A. Inhibiteurs avec une cible cellulaire identifiée

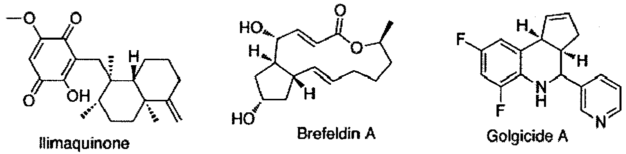

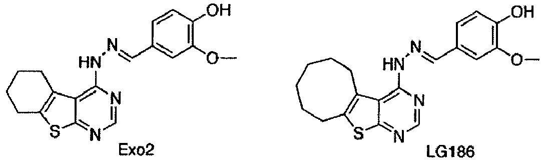

### B. Inhibiteurs dont la cible cellulaire est inconnue

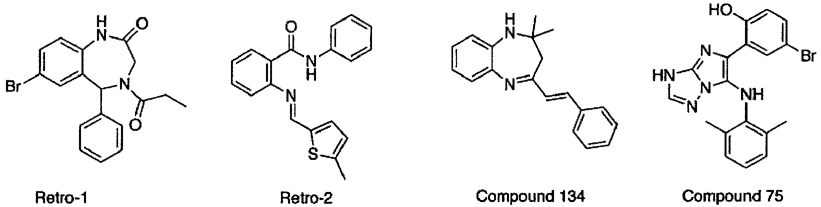

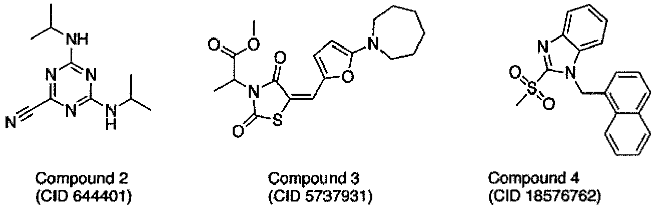

### Petites molécules à activité intracellulaire inhibitrices de l'action de la ricine et/ou des Stx

Très récemment, l'équipe de Park et al. [Park et al. Chemical Structure of Retro-2, a Compound That Protects Cells against Ribosome-Inactivating Proteins. Nature Scientific Reports. 2012. 2; 631] a étudié la structure chimique de Retro-2 et a mis en évidence la cyclisation spontanée de ce composé et montré que c'est sous cette forme cyclisée que Retro-2 exerçait une activité protectrice des cellules contre les toxines.

Dans le cadre de ses recherches sur des composés bloquant le transport rétrograde et plus particulièrement d'études sur des composés dérivés de Retro-2 cyclisé menées parallèlement aux travaux précités, la Demanderesse a identifié une nouvelle famille de composés dérivés de 2,3-dihydroquinazolin-4(1*H*)-one qui présentent une activité biologique supérieure à celle de Retro-2 cyclisée et donc un intérêt marqué pour la prévention et/ou le traitement des intoxications à au moins une toxine à mode d'action intracellulaire utilisant le transport rétrograde pour infecter les cellules eucaryotes de mammifères.

Ainsi, la présente invention se rapporte à des composés de formule générale (I) : 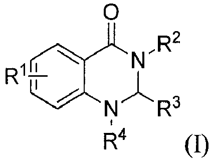 où
**R¹** représente un atome d'hydrogène, un atome d'halogène ou un radical alcoxy de 1 à 3 atomes de carbone ;
**R²** représente :
   - un radical phényle, éventuellement substitué par : un radical phényle, un groupe -CF₃, un atome d'halogène, un groupe -SO₂-phényle ou un groupe -S-X ou
   - O-X, X étant un radical alkyle de 1 à 4 atomes de carbone, de préférence, X est un radical méthyle ;
   - un hétérocycle ou un hétérobicycle de 5 à 10 atomes comprenant un ou deux atomes d'azote, de préférence, un radical pyridine ;
   - un cycle adamantyl, éventuellement substitué par une fonction hydroxyle ;
**R³** représente :
   - un hétérocycle aromatique de 5 à 6 atomes qui peut être choisi parmi un cycle furane, thiophène, pyrrole, pyrroline, pyrrolidine, dioxolane, oxazole, thiazole, imidazole, imidazoline, imidazolidine, pyrazole, isoxazole, isothiazole, pyrane, pyridine, pipéridine, dioxane, morpholine, pyridazine, pyrimidine, pyrazine, de préférence, un cycle thiophène ou pyridine ;
      ledit hétérocycle est substitué par :
      * un radical alkyle de 1 à 3 atomes de carbone, préférentiellement méthyle ;
      * un atome d'halogène ;
      * un radical phényle éventuellement substitué par un radical alcoxy de 1 à 3 atomes de carbone, un groupe -CN, un groupe -NO₂ ou un groupe -COX ou -COOX avec X un radical alkyle de 1 à 4 atomes de carbone ou une combinaison de ces substituants, de préférence, un radical méthyle ;
      * un groupe -SY, Y étant un radical alkyle de 1 à 4 atomes de carbone ou un radical phényle ;
      * un groupe -CN ;
      * un hétérocycle aromatique de 5 ou 6 atomes qui peut être choisi parmi un cycle furane, thiophène, pyrrole, pyrroline, pyrrolidine, dioxolane, oxazole, thiazole, imidazole, imidazoline, imidazolidine, pyrazole, isoxazole, isothiazole, pyrane, pyridine, pipéridine, dioxane, morpholine, pyridazine, pyrimidine, pyrazine, de préférence, ledit hétérocycle de 5 ou 6 atomes est un thiophène, une pyridine, un furane, un thiazole ; éventuellement substitué par au moins un radical alkyle de 1 à 3 atomes de carbone ;
   - un radical phényle ; éventuellement substitué par un radical alcoxy de 1 à 3 atomes de carbone ou un groupe NMe₂ ;
**R⁴** représente un atome d'hydrogène ou un radical méthyle ;
à l'exception du composé tel que R¹ est un atome d'hydrogène, R² un radical phényle, R³ un radical 5-méthylthiophèn-2-yl et R⁴ un atome d'hydrogène,
et leurs sels pharmaceutiquement acceptables.

La présente invention se rapporte au composés de formule générale (I) en tant que tels et également à leur utilisation pour la prévention et/ou le traitement des désordres induits par les toxines à activité intracellulaire utilisant le transport rétrograde.

Par sel pharmaceutiquement acceptable des composés de formule générale (I), on entend les chlorhydrates, bromhydrates, sulfates ou bisulfates, phosphates ou hydrogénophosphates, acétates, oxalates, benzoates, succinates, fumarates, maléates, lactates, citrates, tartrates, gluconates, méthanesulphonates, benzène-sulphonates et paratoluène-sulphonates.

Par atome d'halogène, on entend les éléments chimiques du groupe VII du tableau périodique des éléments, notamment, le fluor, le chlore, le brome et l'iode.

Le terme radical alkyle de 1 à 3 ou 4 atomes de carbone désigne un radical hydrogénocarboné, linéaire ou ramifié ; on peut citer par exemple le méthyle, l'éthyle, le propyle, l'isopropyle ou le tertiobutyle.

Par radical alcoxy de 1 à 3 atomes de carbone, on entend un radical - OCₙH₂ₙ₊₁, n étant un nombre entier compris entre 1 et 3 ; on peut citer par exemple le radical méthoxy, éthoxy, propyloxy, isopropyloxy. De préférence n vaut 1.

Plus particulièrement, les composés de formule générale (I) sont choisis parmi le Tableau I ci-après :

| molécule | composé | structure | Nom |
|---|---|---|---|
| RN-1-013 | 1 | 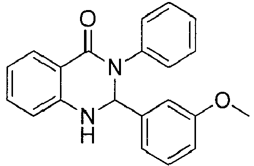 | 2-(3-Methoxyphenyl)-3-phenyl-2,3-dihydroquinazolin-4(1*H*)-one |
| RN-1-019 | 2 | 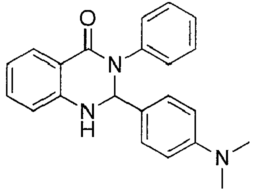 | 2-(4-(Dimethylamino)phenyl)-3-phenyl-2,3-dihydroquinazolin-4(1*H*)-one |
| RN-1-021 | 3 | 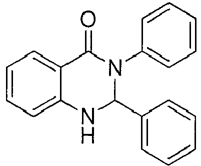 | 2,3-Diphenyl-2,3-dihydroquinazolin-4(1*H*)-one |
| RN-1-027 | 4 | 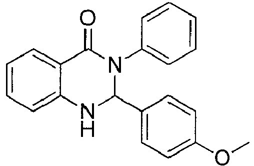 | 2-(4-Methoxyphenyl)-3-phenyl-2,3-dihydroquinazolin-4(1*H*)-one |
| RN-1-066 | 5 | 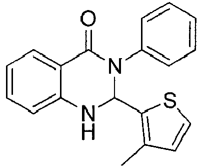 | 2-(3-Methylthiophen-2-yl)-3-phenyl-2,3-dihydroquinazolin-4(1*H*)-one |
| RN-1-067 | 6 | 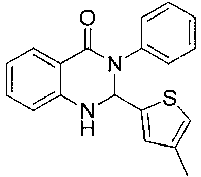 | 2-(4-Methylthiophen-2-yl)-3-phenyl-2,3-dihydroquinazolin-4(1*H*)-one |
| RN-1-068 | 7 | 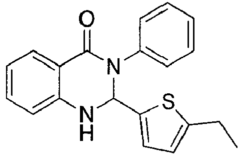 | 2-(5-Ethylthiophen-2-yl)-3-phenyl-2,3-dihydroquinazolin-4(1*H*)-one |
| RN-1-086 | 8 | 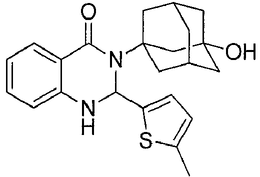 | 3-((5R,7S)-3-Hydroxyadamantan-1-yl)-2-(5-methylthiophen-2-yl)-2,3-dihydroquinazolin-4(1*H*)-one |
| RN-1-100 | 9 | 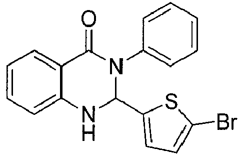 | 2-(5-Bromothiophen-2-yl)-3-phenyl-2,3-dihydroquinazolin-4(1*H*)-one |
| RN-1-118 | 10 | 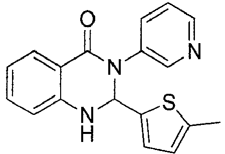 | 2-(5-Methylthiophen-2-yl)-3-(pyridin-3-yl)-2,3-dihydroquinazolin-4(1*H*)-one |
| RN-1-120 | 11 | 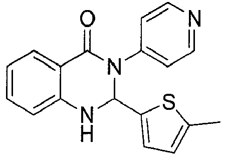 | 2-(5-Methylthiophen-2-yl)-3-(pyridin-4-yl)-2,3-dihydroquinazolin-4(1*H*)-one |
| RN-1-132 | 12 | 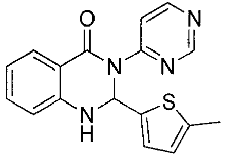 | 2-(5-Methylthiophen-2-yl)-3-(pyrimidin-4-yl)-2,3-dihydroquinazolin-4(1*H*)-one |
| RN-1-148 | 13 | 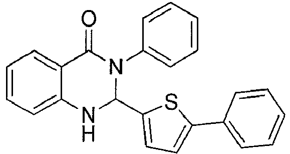 | 3-Phenyl-2-(5-phenylthiophen-2-yl)-2,3-dihydroquinazolin-4(1*H*)-one |
| RN-1-149 | 14 | 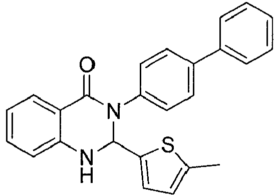 | 3-([1,1'-Biphenyl]-4-yl)-2-(5-methylthiophen-2-yl)-2,3-dihydroquinazolin-4(1*H*)-one |
| RN-1-151 | 15 | 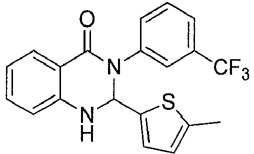 | 2-(5-Methylthiophen-2-yl)-3-(3-(trifluoromethyl)phenyl)-2,3-dihydroquinazolin-4(1*H*)-one |
| RN-1-162 | 16 | 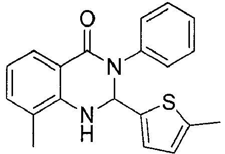 | 8-Methyl-2-(5-methylthiophen-2-yl)-3-phenyl-2,3-dihydroquinazolin-4(1*H*)-one |
| RN-1-173 | 17 | 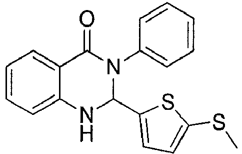 | 2-(5-(Methylthio)thiophen-2-yl)-3-phenyl-2,3-dihydroquinazolin-4(1*H*)-one |
| RN-1-174 | 18 | 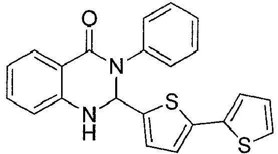 | 2-([2,2'-Bithiophen]-5-yl)-3-phenyl-2,3-dihydroquinazolin-4(1*H*)-one |
| RN-1-175 | 19 | 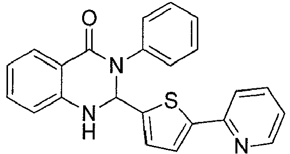 | 3-Phenyl-2-(5-(pyridin-2-yl)thiophen-2-yl)-2,3-dihydroquinazolin-4(1*H*)-one |
| RN-1-176 | 20 | 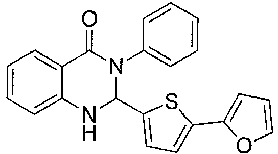 | 2-(5-(Furan-2-yl)thiophen-2-yl)-3-phenyl-2,3-dihydroquinazolin-4(1*H*)-one |
| RN-1-177 | 21 | 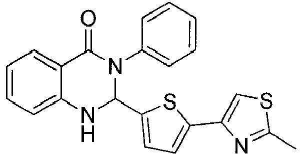 | 2-(5-(2-Methylthiazol-4-yl)thiophen-2-yl)-3-phenyl-2,3-dihydroquinazolin-4(1*H*)-one |
| RN-2-120 | 22 | 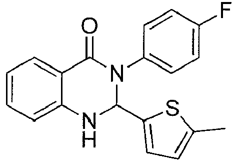 | 3-(4-Fluorophenyl)-2-(5-methylthiophen-2-yl)-2,3-dihydroquinazolin-4(1*H*)-one |
| RN-2-137 | 23 | 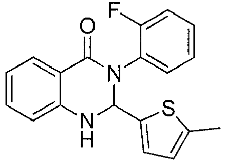 | 3-(2-Fluorophenyl)-2-(5-methylthiophen-2-yl)-2,3-dihydroquinazolin-4(1*H*)-one |
| RN-2-161 | 24 | 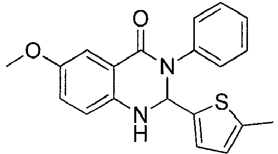 | 6-Methoxy-2-(5-methylthiophen-2-yl)-3-phenyl-2,3-dihydroquinazolin-4(1*H*)-one |
| RN-2-162 | 25 | 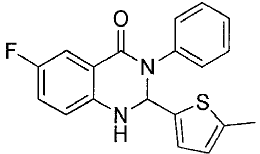 | 6-Fluoro-2-(5-methylthiophen-2-yl)-3-phenyl-2,3-dihydroquinazolin-4(1*H*)-one |
| RN-2-163 | 26 | 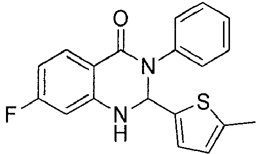 | 7-Fluoro-2-(5-methylthiophen-2-yl)-3-phenyl-2,3-dihydroquinazolin-4(1*H*)-one |
| RN-2-177 | 27 | 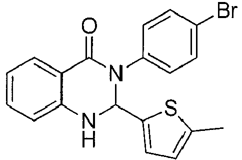 | 3-(4-Bromophenyl)-2-(5-methylthiophen-2-yl)-2,3-dihydroquinazolin-4(1*H*)-one |
| RN-2-178 | 28 | 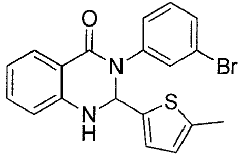 | 3-(3-Bromophenyl)-2-(5-methylthiophen-2-yl)-2,3-dihydroquinazolin-4(1*H*)-one |
| RN-2-180 | 29 | 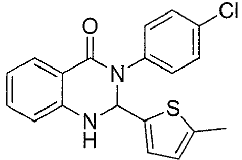 | 3-(4-Chlorophenyl)-2-(5-methylthiophen-2-yl)-2,3-dihydroquinazolin-4(1*H*)-one |
| RN-2-181 | 30 | 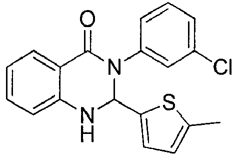 | 3-(3-Chlorophenyl)-2-(5-methylthiophen-2-yl)-2,3-dihydroquinazolin-4(1*H*)-one |
| RN-2-183 | 31 | 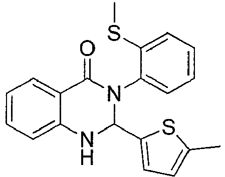 | 3-(2-(Methylthio)phenyl)-2-(5-methylthiophen-2-yl)-2,3-dihydroquinazolin-4(1*H*)-one |
| RN-2-182 | 32 | 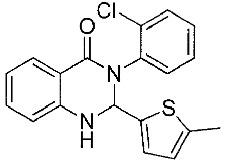 | 3-(2-Chlorophenyl)-2-(5-methylthiophen-2-yl)-2,3-dihydroquinazolin-4(1*H*)-one |
| RN-2-199 | 33 | 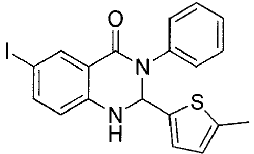 | 6-Iodo-2-(5-methylthiophen-2-yl)-3-phenyl-2,3-dihydroquinazolin-4(1*H*)-one |
| RN-3-121 | 34 | 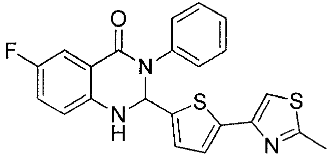 | 6-fluoro-2-(5-(2-methylthiazol-4-yl)thiophen-2-yl)-3-phenyl-2,3-dihydroquinazolin-4(1H)-one |
| RN-1-181 | 35 | 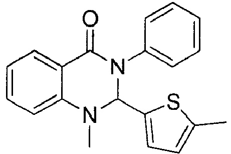 | 1-Methyl-2-(5-methylthiophen-2-yl)-3-phenyl-2,3-dihydroquinazolin-4(1*H*)-one |
| RN-1-186 | 36 | 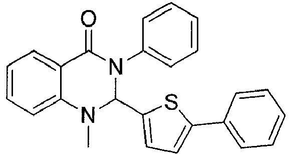 | 1-Methyl-3-phenyl-2-(5-phenylthiophen-2-yl)-2,3-dihydroquinazolin-4(1*H*)-one |
| RN-1-192 | 37 | 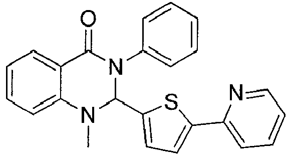 | 1-Methyl-3-phenyl-2-(5-(pyridin-2-yl)thiophen-2-yl)-2,3-dihydroquinazolin-4(1*H*)-one |
| RN-1-196 | 38 | 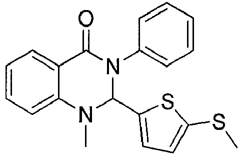 | 1-Methyl-2-(5-(methylthio)thiophen-2-yl)-3-phenyl-2,3-dihydroquinazolin-4(1*H*)-one |
| RN-1-197 | 39 | 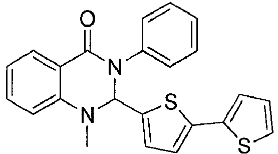 | 2-([2,2'-Bithiophen]-5-yl)-1-methyl-3-phenyl-2,3-dihydroquinazolin-4(1*H*)-one |
| RN-2-001 | 40 | 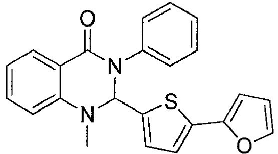 | 2-(5-(Furan-2-yl)thiophen-2-yl)-1-methyl-3-phenyl-2,3-dihydroquinazolin-4(1*H*)-one |
| RN-2-005 | 41 | 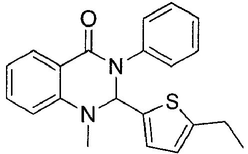 | 2-(5-Ethylthiophen-2-yl)-1-methyl-3-phenyl-2,3-dihydroquinazolin-4(1*H*)-one |
| RN-2-015 | 42 | 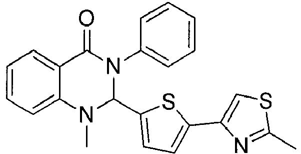 | 1-Methyl-2-(5-(2-methylthiazol-4-yl)thiophen-2-yl)-3-phenyl-2,3-dihydroquinazolin-4(1*H*)-one |
| RN-2-016 | 43 | 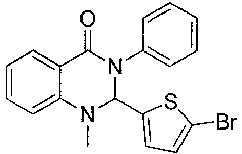 | 2-(5-Bromothiophen-2-yl)-1-methyl-3-phenyl-2,3-dihydroquinazolin-4(1*H*)-one |
| RN-2-019 | 44 | 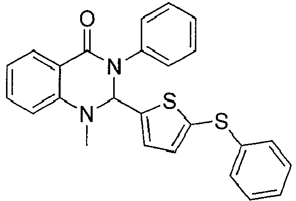 | 1-Methyl-3-phenyl-2-(5-(phenylthio)thiophen-2-yl)-2,3-dihydroquinazolin-4(1*H*)-one |
| RN-2-029 | 45 | 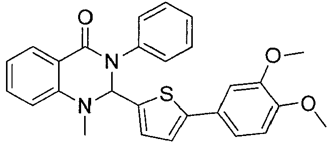 | 2-(5-(3,4-Dimethoxyphenyl)thiophen-2-yl)-1-methyl-3-phenyl-2,3-dihydroquinazolin-4(1*H*)-one |
| RN-2-032 | 46 | 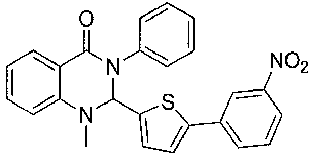 | 1-Methyl-2-(5-(3-nitrophenyl)thiophen-2-yl)-3-phenyl-2,3-dihydroquinazolin-4(1*H*)-one |
| RN-2-034 | 47 | 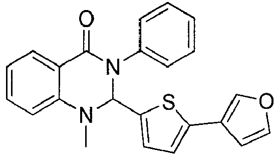 | 2-(5-(Furan-3-yl)thiophen-2-yl)-1-methyl-3-phenyl-2,3-dihydroquinazolin-4(1*H*)-one |
| RN-2-049 | 48 | 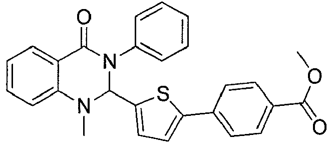 | Methyl 4-(5-(1-methyl-4-oxo-3-phenyl-1,2,3,4-tetrahydroquinazolin-2-yl)thiophen-2-yl)benzoate |
| RN-2-050 | 49 | 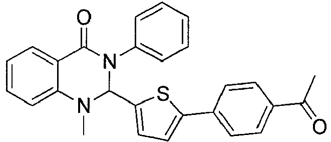 | 2-(5-(4-Acetylphenyl)thiophen-2-yl)-1-methyl-3-phenyl-2,3-dihydroquinazolin-4(1*H*)-one |
| RN-2-053 | 50 | 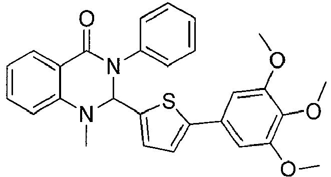 | 1-Methyl-3-phenyl-2-(5-(3,4,5-trimethoxyphenyl)thiophen-2-yl)-2,3-dihydroquinazolin-4(1*H*)-one |
| RN-2-057 | 51 | 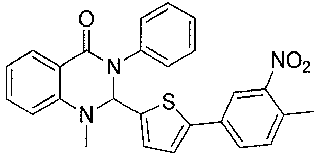 | 1-methyl-2-(5-(4-methyl-3-nitrophenyl)thiophen-2-yl)-3-phenyl-2,3-dihydroquinazolin-4(1H)-one |
| RN-2-059 | 52 | 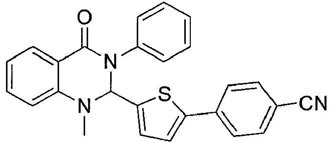 | 4-(5-(1-Methyl-4-oxo-3-phenyl-1,2,3,4-tetrahydroquinazolin-2-yl)thiophen-2-yl)benzonitrile |
| RN-3-012 | 53 | 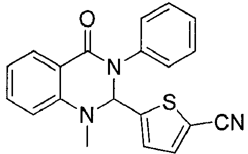 | 5-(1-Methyl-4-oxo-3-phenyl-1,2,3,4-tetrahydroquinazolin-2-yl)thiophene-2-carbonitrile |
| RN-3-066 | 54 | 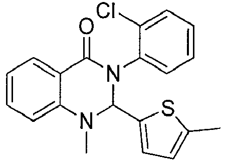 | 3-(2-chlorophenyl)-1-methyl-2-(5-methylthiophen-2-yl)-2,3-dihydroquinazolin-4(1H)-one |
| RN-3-067 | 55 | 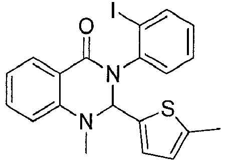 | 3-(2-iodophenyl)-1-methyl-2-(5-methylthiophen-2-yl)-2,3-dihydroquinazolin-4(1H)-one |
| RN-3-068 | 56 | 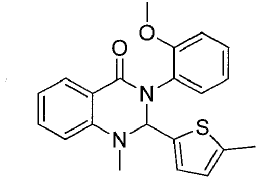 | 3-(2-methoxyphenyl)-1-methyl-2-(5-methylthiophen-2-yl)-2,3-dihydroquinazolin-4(1H)-one |
| RN-3-069 | 57 | 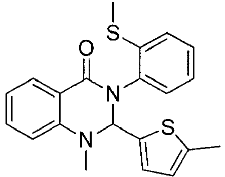 | 1-methyl-3-(2-(methylthio)phenyl)-2-(5-methylthiophen-2-yl)-2,3-dihydroquinazolin-4(1H)-one |
| RN-3-070 | 58 | 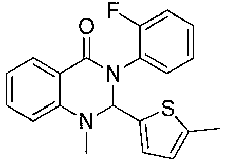 | 3-(2-fluorophenyl)-1-methyl-2-(5-methylthiophen-2-yl)-2,3-dihydroquinazolin-4(1H)-one |
| RN-3-089 | 59 | 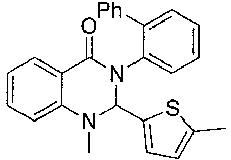 | 3-([1,1'-biphenyl]-2-yl)-1-methyl-2-(5-methylthiophen-2-yl)-2,3-dihydroquinazolin-4(1H)-one |
| RN-3-098 | 60 | 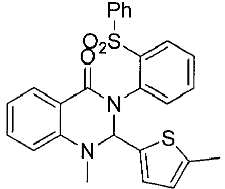 | 1-methyl-2-(5-methylthiophen-2-yl)-3-(2-(phenylsulfonyl)phenyl)-2,3-dihydroquinazolin-4(1H)-one |
| RN-3-122 | 61 | 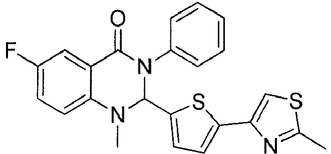 | 6-fluoro-1-methyl-2-(5-(2-methylthiazol-4-yl)thiophen-2-yl)-3-phenyl-2,3-dihydroquinazolin-4(1H)-one |

Selon un mode de réalisation préféré de l'invention, les composés de formule générale (I) sont tels que :
- R¹ est un atome d'hydrogène ou un atome de fluor ;
- R² est un cycle phényle éventuellement substitué par : un atome de chlore, d'iode ou de fluor, un radical -OCH₃, -SCH₃, -SO₂-phényle ou un phényle ;
- R³ est un radical thiophène substitué par un radical méthyle, éthyle, phényle, -SCH₃, 2-thiophene, 2-furane, 3-furane, 2-pyridine, 4-(2-Me thiazole), un atome de brome, S-phényle et
- R⁴ est un atome d'hydrogène ou un radical méthyle ;
à l'exception du composé tel que R¹ est un atome d'hydrogène, R² un radical phényle, R³ un radical 5-méthylthiophèn-2-yl et R⁴ un atome d'hydrogène,
et leur sels pharmaceutiquement acceptables.

Les composés préférés selon l'invention sont ceux choisis parmi 7, 9, 13, 17, 18, 19, 20, 21, 25, 31, 32, 34, 35, 36, 37, 38, 39, 40, 41, 42, 43, 44, 47, 54, 55, 56, 57, 58,59,60,61.

Les composés de formule générale (I) pour lesquels R⁴ est un atome d'hydrogène sont obtenus à partir des composés de type imine analogues de Retro-2 (voir la Demande EP 2 145 873) par cyclisation en milieu basique (voie A) ou directement par réaction de l'anthranilamide avec l'aldéhyde (voie B) :

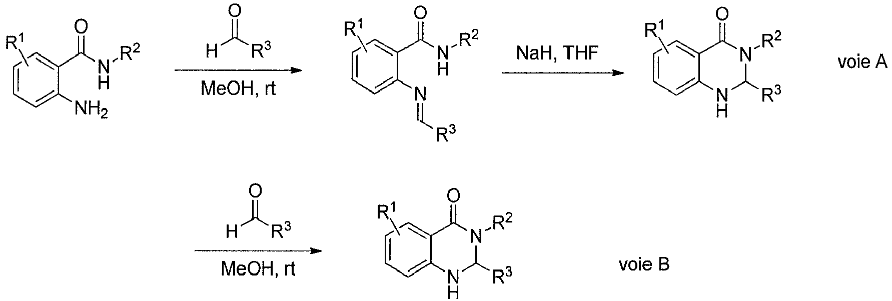

Les composés de formule générale telle que R⁴ est un substituant méthyle comme représenté ci-contre : 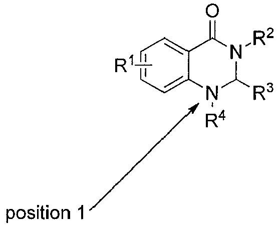 sont obtenus par déprotonation de l'amine en position 1 et piégeage de l'anion par de l'iodure de méthyle. 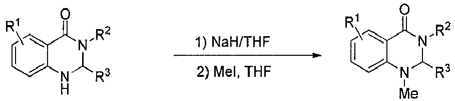 où R¹, R² et R³ sont définis comme précédemment.

Certains des composés selon l'invention, en particulier, les composés 45, 46, 47, 48, 49, 50, 51 et 52 ont été synthétisés par la voie décrite ci-après : 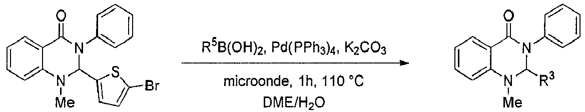 ou par couplage du composé 43 avec PhSH en présence de Pd₂dba₃ (0.1 équiv.), dppf (0.15 équiv.) et *t*BuOK (2 équiv.) dans le dioxane, 1h à 140 °C au micro-onde (composé 44) ou ZnCN₂ en présence de Pd₂dba₃ (0.05 équiv.), dppf (0.1 équiv.) dans le DMF, 1h à 150 °C au micro-onde (composé 53).

Les composés selon l'invention sont des substances pharmacologiquement actives et trouvent leur intérêt grâce à leur effet inhibiteur des toxines à mode d'action intracellulaire, en particulier, de la ricine.

L'utilisation des composés de formule générale (I) est particulièrement avantageuse pour prévenir et/ou traiter les désordres provoqués par les toxines à mode d'action intracellulaire utilisant le transport rétrograde pour intoxiquer les cellules eucaryotes de mammifères.

Plus spécifiquement, les toxines à mode d'action intracellulaire sont : notamment la ricine (produite dans les graines de la plante *Ricinus communis*), la toxine de Shiga et les toxines Shiga-like (Stxs) produite par *Shigella dysenteriae* (Stx) et *E*. *coli* (Stx1 et Stx2), la toxine cholérique (Ctx de *Vibrio cholerae* responsable du choléra), la toxine pertussique (*Bordetella pertussis* agent de la coqueluche), la cytotoxine subtilase et l'entérotoxine thermolabile *(E. coli*),

L'utilisation des composés selon l'invention s'avère efficace que le sujet ingère ou inhale la toxine, ou encore que la toxine lui soit injectée.

Ainsi les composés selon l'invention pourront être utilisés pour prévenir et/ou traiter l'intoxication des cellules eucaryotes mammifères par des toxines à mode d'action intracellulaire.

De façon concrète, une toxine comme la ricine, lorsqu'elle est inhalée, conduit à des signes d'irritation oculaire (sensation de brûlure, larmoiement, conjonctivite plus ou moins sévère) et pharyngée ainsi qu'une irritation respiratoire plus ou moins marquée : toux, dyspnée, oedème pulmonaire pouvant conduire à un syndrome de détresse respiratoire aigu (SDRA). Il est à noter qu'il existe un risque de réaction anaphylactique. La dose létale est de 1 mg/kg de poids corporel (Ministère de la santé, France).

Ainsi, l'invention se rapporte à l'utilisation d'un composé de formule générale (I) pour prévenir et/ou traiter les intoxications à la ricine ou à d'autres toxines à mode d'action intracellulaire et protéger contre ces intoxications les cellules eucaryotes, notamment, épithéliales, oculaires, pharyngiennes, trachéales, bronchiques, cutanées, musculaires, en particulier, des cellules épithéliales pulmonaires et digestives, de préférence intestinales, de mammifères, de préférence de l'homme.

L'invention se rapporte également à des compositions pharmaceutiques ou médicament comprenant un ou plusieurs composés de formule générale (I) dans un véhicule pharmaceutiquement acceptable.

Par pharmaceutiquement acceptable, on entend compatible avec une administration à un sujet, de préférence un mammifère, par toute voie d'administration.

L'homme du métier saura adapter la formulation des composés de formule générale (I) selon leurs propriétés physico-chimiques et leur voie d'administration.

Le médicament pourra être administré par voie orale (notamment dans la cavité buccale ou en administration sublinguale), parentérale, pulmonaire, oculaire, nasale.... D'autres modes d'administration envisageable comprennent la voie intra-péritonéale (i.p), intraveineuse (i.v.), sous-cutanée (s.c.), intramusculaire (i.m.), transcutanée, transdermale, intracathécale, épidurale, sous-mucosale, par inhalation nasale ou pulmonaire.

Les modes d'administration des composés de formule générale (I) préférés sont ceux utilisant les voies aériennes (inhalation nasale ou pulmonaire), orale (ingestion), parentérale ou locale (topique).

La présente invention se rapporte ainsi à des compositions, en particulier des compositions pharmaceutiques, comprenant au moins un composé de formule générale (I) selon l'invention et, éventuellement, un véhicule pharmaceutiquement acceptable, des excipients stabilisants et des adjuvants classiquement utilisés.

La quantité de composé de formule générale (I) à administrer au mammifère dépend de l'activité propre de ce composé, activité qui peut être mesurée par des moyens qui sont exposés dans les exemples. Cette quantité dépend également de la gravité de la pathologie à traiter, notamment de la quantité de toxine absorbée et de la voie par laquelle elle l'a été, elle dépend enfin de l'âge et du poids de l'individu à traiter.

Outre les dispositions qui précèdent, l'invention comprend encore d'autres dispositions qui ressortiront de la description qui va suivre, qui se réfèrent à des exemples de mise en oeuvre de la présente invention, ainsi qu'aux figures annexées dans lesquelles :
La **Figure 1** représente les structures cristallographiques de la ricine (A, dont le nom dans la base de données des structures cristallographiques est pdb 2AAI) et de la Stx2 (B, pdb 1R4P).
La **Figure 2** illustre schématiquement l'entrée et le routage intracellulaire de la ricine (A) et des toxines de Shiga (B).
La **Figure 3** est une représentation schématique du test cellulaire mis en oeuvre dans la partie expérimentale.

### Exemples

### I. Synthèse de composés selon l'invention

Les composés de formule générale (I) qui suivent sont préparés selon le schéma réactionnel qui suit :

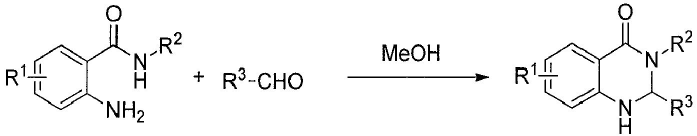

Protocole : dans un tube scellé sont ajoutés le 2-amino benzamide (4.71 mmol), du THF (23 mL, 0.2M) puis l'aldéhyde (4.71 mmol) et de l'acide para-toluène sulfonique (10 mol %, 0.5 mmol). La solution est chauffée à 75 °C et agitée à cette température jusqu'à disparition complète des produits de départ (suivi par chromatographie sur couche mince, typiquement une nuit de temps de réaction). Après refroidissement à température ambiante, de la silice est ajoutée et le THF est évaporé. Une purification par chromatographie sur gel de silice suivie d'une évaporation fournit les produits attendus sous forme de solides.

**Tableau II. Molécules cycliques de type 2,3-dihydroquinazolin-4(1H)-one**

| molécule | composé | Nom | rendement | LC/ MS | ¹H | ¹³C |
|---|---|---|---|---|---|---|
| RN-1-013 | 1 | 2-(3-Methoxyphenyl)-3-phenyl-2,3-dihydroqumazolin-4(1*H*)-one | 88% | x | x | x |
| RN-1-019 | 2 | 2-(4-(Dimethylamino)phenyl)-3-phenyl-2,3-dihydroquinazolin-4(1*H*)-one | 77% | x | x | x |
| RN-1-021 | 3 | 2,3-Diphenyl-2,3-dihydroquinazolin-4(1*H*)-one | 84% | x | x | x |
| RN-1-027 | 4 | 2-(4-Methoxyphenyl)-3-phenyl-2,3-dihydroquinazolin-4(1*H*)-one | 78% | x | x | x |
| RN-1-066 | 5 | 2-(3-Methylthiophen-2-yl)-3-phenyl-2,3-dihydroquinazolin-4(1*H*)-one | 85% | x | x | x |
| RN-1-067 | 6 | 2-(4-Methylthiophen-2-yl)-3-phenyl-2,3-dihydroquinazolin-4(1*H*)-one | 65% | x | x | x |
| RN-1-068 | 7 | 2-(5-Ethylthiophen-2-yl)-3-phenyl-2,3-dihydroquinazolin-4(1*H*)-one | 77% | x | x | x |
| RN-1-086 | 8 | 3-((5R,7S)-3-Hydroxyadamantan-1-yl)-2-(5-methylthiophen-2-yl)-2,3-dihydroquinazolin-4(1*H*)-one | 81% | x | x | x |
| RN-1-100 | 9 | 2-(5-Bromothiophen-2-yl)-3-phenyl-2,3-dihydroquinazolin-4(1*H*)-one | 91% | x | x | x |
| RN-1-118 | 10 | 2-(5-Methylthiophen-2-yl)-3-(pyridin-3-yl)-2,3-dihydroquinazolin-4(1*H*)-one | 34% | x | x | x |
| RN-1-120 | 11 | 2-(5-Methylthiophen-2-yl)-3-(pyridin-4-yl)-2,3-dihydroquinazolin-4(1*H*)-one | 64% | x | x | x |
| RN-1-132 | 12 | 2-(5-Methylthiophen-2-yl)-3-(pyrimidin-4-yl)-2,3-dihydroquinazolin-4(1*H*)-one | 53% | x | x | x |
| RN-1-148 | 13 | 3-Phenyl-2-(5-phenylthiophen-2-yl)-2,3-dihydroquinazolin-4(1*H*)-one | 86% | x | x | x |
| RN-1-149 | 14 | 3-([1,1'-Biphenyl]-4-yl)-2-(5-methylthiophen-2-yl)-2,3-dihydroquinazolin-4(1*H*)-one | 73% | x | x | x |
| RN-1-151 | 15 | 2-(5-Methylthiophen-2-yl)-3-(3-(trifluoromethyl)phenyl)-2,3-dihydroquinazolin-4(1*H*)-one | 68% | x | x | x |
| RN-1-162 | 16 | 8-Methyl-2-(5-methylthiophen-2-yl)-3-phenyl-2,3-dihydroquinazolin-4(1*H*)-one | 99% | x | x | x |
| RN-1-173 | 17 | 2-(5-(Methylthio)thiophen-2-yl)-3-phenyl-2,3-dihydroquinazolin-4(1*H*)-one | 82% | x | x | x |
| RN-1-174 | 18 | 2-([2,2'-Bithiophen]-5-yl)-3-phenyl-2,3-dihydroquinazolin-4(1*H*)-one | 86% | x | x | x |
| RN-1-175 | 19 | 3-Phenyl-2-(5-(pyridin-2-yl)thiophen-2-yl)-2,3-dihydroquinazolin-4(1*H*)-one | 72% | x | x | x |
| RN-1-176 | 20 | 2-(5-(Furan-2-yl)thiophen-2-yl)-3-phenyl-2,3-dihydroquinazolin-4(1*H*)-one | 64% | x | x | x |
| RN-1-177 | 21 | 2-(5-(2-Methylthiazol-4-yl)thiophen-2-yl)-3-phenyl-2,3-dihydroquinazolin-4(1*H*)-one | 68% | x | x | x |
| RN-2-120 | 22 | 3-(4-Fluorophenyl)-2-(5-methylthiophen-2-yl)-2,3-dihydroquinazolin-4(1*H*)-one | 80% | x | x | x |
| RN-2-137 | 23 | 3-(2-Fluorophenyl)-2-(5-methylthiophen-2-yl)-2,3-dihydroquinazolin-4(1*H*)-one | 93% | x | x | x |
| RN-2-161 | 24 | 6-Methoxy-2-(5-methylthiophen-2-yl)-3-phenyl-2,3-dihydroquinazolin-4(1*H*)-one | 89% | x | x | x |
| RN-2-162 | 25 | 6-Fluoro-2-(5-methylthiophen-2-yl)-3-phenyl-2,3-dihydroquinazolin-4(1*H*)-one | 70% | x | x | x |
| RN-2-163 | 26 | 7-Fluoro-2-(5-methylthiophen-2-yl)-3-phenyl-2,3-dihydroquinazolin-4(1*H*)-one | 80% | x | x | x |
| RN-2-177 | 27 | 3-(4-Bromophenyl)-2-(5-methylthiophen-2-yl)-2,3-dihydroquinazolin-4(1*H*)-one | 80% | x | x | x |
| RN-2-178 | 28 | 3-(3-Bromophenyl)-2-(5-methylthiophen-2-yl)-2,3-dihydroquyinazolin-4(1*H*)-one | 91% | x | x | x |
| RN-2-180 | 29 | 3-(4-Chlorophenyl)-2-(5-methylthiophen-2-yl)-2,3-dihydroquinazolin-4(1*H*)-one | 89% | x | x | x |
| RN-2-181 | 30 | 3-(3-Chlorophenyl)-2-(5-methylthiophen-2-yl)-2,3-dihydroquinazolin-4(1*H*)-one | 91% | x | x | x |
| RN-2-183 | 31 | 3-(2-(Methylthio)phenyl)-2-(5-methylthiophen-2-yl)-2,3-dihydroquinazolin-4(1*H*)-one | 79% | x | x | x |
| RN-2-182 | 32 | 3-(2-Chlorophenyl)-2-(5-methylthiophen-2-yl)-2,3-dihydroquinazolin-4(1*H*)-one | 92% | x | x | x |
| RN-2-199 | 33 | 6-Iodo-2-(5-methylthiophen-2-yl)-3-phenyl-2,3-dihydroquinazolin-4(1*H*)-one | 92% | | x | |
| RN-3-121 | 34 | 6-fluoro-2-(5-(2-methylthiazol-4-yl)thiophen-2-yl)-3-phenyl-2,3-dihydroquinazolin-4(1H)-one | 36% | x | x | x |

Synthèse des composés de formule générale (I) pour lesquels R⁴ est un radical méthyle :

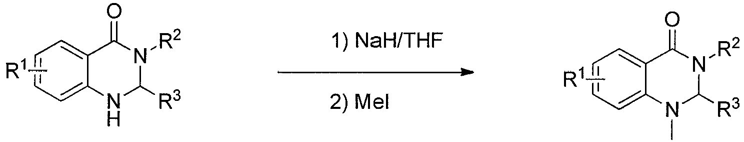

Protocole : à une suspension de NaH (1.5 équiv.) dans du tétrahydrofuranne anhydre (0.3M) est ajouté goutte à goutte une solution de N-H tetrahydroquinazolin-4-ones (1 équiv.) dans du THF anhydre (0.5M) à 0°C. Après 30min, de l'iodométhane (1.1 équiv.) est ajouté goutte à goutte. Après 10 minutes de réaction, la solution est réchauffée à température ambiante et le milieu réactionnel est agité pendant 3h. La réaction est stoppée par ajout d'une solution saturée de NaHCO₃. La phase aqueuse est extraite par du CH₂Cl₂ (3 x 50 mL). les phases organiques sont réunies puis lavées par une solution 1M solution d'HCl (2 x 10 mL), séchées sur Na₂SO₄ anhydre, filtrée et concentrée à l'évaporateur rotatif. Une purification par chromatographie sur gel de silice fournit les produits attendus sous forme de solides.

**Tableau III**

| molécule | composé | nom | rendement | LC/MS | ¹H | ¹³C |
|---|---|---|---|---|---|---|
| RN-1-181 | 35 | 1-Methyl-2-(5-methylthiophen-2-yl)-3-phenyl-2,3-dihydroquinazolin-4(1*H*)-one | 85% à partir de RN-1-001 | x | x | x |
| RN-1-186 | 36 | 1-Methyl-3-phenyl-2-(5-phenylthiophen-2-yl)-2,3-dihydroquinazolin-4(1*H*)-one | 69% à partir de RN-1-069 | x | x | x |
| RN-1-192 | 37 | 1-Methyl-3-phenyl-2-(5-(pyridin-2-yl)thiophen-2-yl)-2,3-dihydroquinazolin-4(1H)-one | 97% à partir de RN-1-101 | x | x | x |
| RN-1-196 | 38 | 1-Methyl-2-(5-(methylthio)thiophen-2-yl)-3-phenyl-2,3-dihydroquinazolin-4(1*H*)-one | 91% à partir de RN-1-077 | x | x | x |
| RN-1-197 | 39 | 2-([2,2'-Bithiophen]-5-yl)-1-methyl-3-phenyl-2,3-dihydroquinazolin-4(1*H*)-one | 95% à partir de RN-1-080 | x | x | x |
| RN-2-001 | 40 | 2-(5-(Furan-2-yl)thiophen-2-yl)-1-methyl-3-phenyl-2,3-dihydroquinazolin-4(1*H*)-one | 97% à partir de RN-1-104 | x | x | x |
| RN-2-005 | 41 | 2-(5-Ethylthiophen-2-yl)-1-methyl-3-phenyl-2,3-dihydroquinazolin-4(1*H*)-one | 84% à partir de RN-1-068 | x | x | x |
| RN-2-015 | 42 | 1-Methyl-2-(5-(2-methylthiazol-4-yl)thiophen-2-yl)-3-phenyl-2,3-dihydroquinazolin-4(1*H*)-one | 83% à partir de RN-1-105 | x | x | x |
| RN-2-016 | 43 | 2-(5-Bromothiophen-2-yl)-1-methyl-3-phenyl-2,3-dihydroquinazolin-4(1*H*)-one | 85% à partir de RN-1-100 | x | x | x |
| RN-2-019 | 44 | 1-Methyl-3-phenyl-2-(5-(phenylthio)thiophen-2-yl)-2,3-dihydroquinazolin-4(1*H*)-one | 91% à partir de RN-2-016 | x | x | x |
| RN-2-029 | 45 | 2-(5-(3,4-Dimethoxyphenyl)thiophen-2-yl)-1-methyl-3-phenyl-2,3-dihydroquinazolin-4(1*H*)-one | 88% à partir de RN-2-016 | x | x | x |
| RN-2-032 | 46 | 1-Methyl-2-(5-(3-nitrophenyl)thiophen-2-yl)-3-phenyl-2,3-dihydroquinazolin-4(1*H*)-one | 90% à partir de RN-2-016 | x | x | x |
| RN-2-034 | 47 | 2-(5-(Furan-3-yl)thiophen-2-yl)-1-methyl-3-phenyl-2,3-dihydroquinazolin-4(1*H*)-one | 80% à partir de RN-2-016 | x | x | x |
| RN-2-049 | 48 | Methyl 4-(5-(1-methyl-4-oxo-3-phenyl-1,2,3,4-tetrahydroquinazolin-2-yl)thiophen-2-yl)benzoate | 80% à partir de RN-2-016 | x | x | x |
| RN-2-050 | 49 | 2-(5-(4-Acetylphenyl)thiophen-2-yl)-1-methyl-3-phenyl-2,3-dihydroquinazolin-4(1*H*)-one | 89% à partir de RN-2-016 | x | x | x |
| RN-2-053 | 50 | 1-Methyl-3-phenyl-2-(5-(3,4,5-trimethoxyphenyl)thiophen-2-yl)-2,3-dihydroquinazolin-4(1*H*)-one | 73% à partir de RN-2-016 | x | x | x |
| RN-2-057 | 51 | 1-methyl-2-(5-(4-methyl-3-nitrophenyl)thiophen-2-yl)-3-phenyl-2,3-dihydroquinazolin-4(1H)-one | 76% à partir de RN-2-016 | x | x | x |
| RN-2-059 | 52 | 4-(5-(1-Methyl-4-oxo-3-phenyl-1,2,3,4-tetrahydroquinazolin-2-yl)thiophen-2-yl)benzonitrile | 78% à partir de RN-2-016 | x | x | x |
| RN-3-012 | 53 | 5-(1-Methyl-4-oxo-3-phenyl-1,2,3,4-tetrahydroquinazolin-2-yl)thiophene-2-carbonitrile | 68% à partir de RN-2-016 | x | | |
| RN-3-066 | 54 | 3-(2-chlorophenyl)-1-methyl-2-(5-methylthiophen-2-yl)-2,3-dihydroquinazolin-4(1H)-one | 67% | x | x | x |
| RN-3-067 | 55 | 3-(2-iodophenyl)-1-methyl-2-(5-methylthiophen-2-yl)-2,3-dihydroquinazolin-4(1H)-one | 46% | x | x | x |
| RN-3-068 | 56 | 3-(2-methoxyphenyl)-1-methyl-2-(5-methylthiophen-2-yl)-2,3-dihydroquinazolin-4(1H)-one | 28% | x | x | x |
| RN-3-069 | 57 | 1-methyl-3-(2-(methylthio)phenyl)-2-(5-methylthiophen-2-yl)-2,3-dihydroquinazolin-4(1H)-one | 73% | x | x | x |
| RN-3-070 | 58 | 3-(2-fluorophenyl)-1-methyl-2-(5-methylthiophen-2-yl)-2,3-dihydroquinazolin-4(1H)-one | 51% | x | x | x |
| RN-3-089 | 59 | 3-([1,1'-biphenyl]-2-yl)-1-methyl-2-(5-methylthiophen-2-yl)-2,3-dihydroquinazolin-4(1H)-one | 74% | x | x | x |
| RN-3-098 | 60 | 1-methyl-2-(5-methylthiophen-2-yl)-3-(2-(phenylsulfonyl)phenyl)-2,3-dihydroquinazolin-4(1H)-one | 65% | x | x | x |
| RN-3-122 | 61 | 6-fluoro-1-methyl-2-(5-(2-methylthiazol-4-yl)thiophen-2-yl)-3-phenyl-2,3-dihydroquinazolin-4(1H)-one | 71% | x | x | x |

### II. Mesure de l'activité protectrice des composés vis-à-vis des toxines sur cellules

### Protocole expérimental

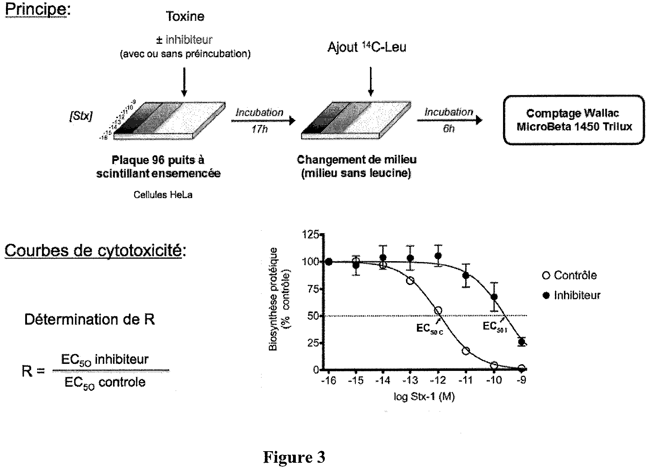

Les composés ont été testés soit sur cellules A549 (cellules épithéliales pulmonaires humaines) soit sur cellules HeLa (cellules de cancer utérin humain), respectivement contre la ricine et contre les toxines de Shiga (Stx-1 et/ou Stx-2). Les cellules humaines sont cultivées à 37°C dans une atmosphère contenant 5% de CO₂ sur des flasques de culture de 150 cm² dans du milieu DMEM (Dulbecco's Modified Eagle Medium) contenant 100 U/mL de pénicilline et 100 µg/mL de streptomycine. Les cellules sont ensemencées à une densité de 50000 cellules par puits dans des plaques 96 puits à fond en scintillant solide Cytostar-T (**Figure 3**). Les cellules (100 µL dans du DMEM complet: DMEM + 10% de sérum de veau foetal, SVF) sont pré-incubées ou non avec les inhibiteurs (50 µL; différentes concentrations, pré-incubation de 3 h). Le milieu complet complémenté de toxine (50 µL, gamme de concentration variable) est ensuite ajouté à chaque puits. Après une incubation de 20 h, le milieu (200 µL) est éliminé et remplacé par un milieu DMEM sans leucine (Eurobio) contenant 10% de SVF et 0,5 µCi/mL de ¹⁴C-leucine (GE). Après une incubation de 7 h à 37°C, l'incorporation de radioactivité par les cellules est déterminée par lecture des plaques par un compteur à scintillation Wallac 1450 Microbeta trilux (PE).

Comme ces toxines bloquent la synthèse des protéines, les cellules affectées ne sont plus capables d'incorporer la leucine radiomarquée. Par contre, les cellules traitées par des inhibiteurs synthétisent toujours des protéines et incorporent donc l'acide aminé radiomarqué. Comme les cellules concentrent le radioélément suffisamment près du fond du puits, cela entraine une excitation du scintillant contenu dans les plaques et conduit à l'émission de photons détectée par le compteur à scintillation (mesure en coups par minutes, cpm). Ces données sont ensuite exprimées en pourcentage de synthèse de protéine par les cellules. Les courbes de cytotoxicité peuvent ainsi être tracées (R, **Figure 3**) sans inhibiteur (ronds blancs) ou en présence d'un inhibiteur (ronds noirs). L'analyse des données par régression non linéaire permet d'estimer l'EC₅₀, soit la concentration efficace pour laquelle on observe 50 % d'assimilation de leucine radioactive ce qui correspond à 50% de cellules viables. Plus la valeur de l'EC₅₀ est élevée, plus la protection cellulaire est importante car il faut alors une concentration plus élevée de toxine pour générer la même cytotoxicité. Il est ainsi possible de déterminer l'efficacité des inhibiteurs en calculant le ratio des EC₅₀ (R, *cf. **Schéma 6***)**.** Plus la valeur est élevée (> 1), plus la protection des cellules est importante.

### Résultats

Le Tableau IV ci-dessous présente les résultats sous la forme d'un indice de protection (ratio EC₅₀ composé / EC₅₀ ricine) : plus il est élevé, plus les cellules sont protégées contre l'action de la ricine (effet protecteur si > 1).

**Tableau IV. Evaluation des activités biologiques des composés selon l'invention.**

| **molécule** | **composé** | **R (ricine)** | **R (Stx)** |
|---|---|---|---|
| Contrôle | Retro-2 | 2,7 | 25 |
| RN-1-068 | 7 | 2,6 | 120 |
| RN-1-100 | 9 | 1,8 | 27,9 |
| RN-1-148 | 13 | 4,2 | 153 |
| RN-1-173 | 17 | 2,8 | 43 |
| RN-1-174 | 18 | 5,6 | 50 |
| RN-1-175 | 19 | 3,1 | 46 |
| RN-1-176 | 20 | 0,7 | 41 |
| RN-1-177 | 21 | 7,3 | 109 |
| RN-2-162 | 25 | - | 133 |
| RN-2-183 | 31 | - | 99 |
| RN-2-182 | 32 | - | 68 |
| RN-3-121 | 34 | - | 295 |
| RN-1-181 | 35 | 6,4 | 80 |
| RN-1-186 | 36 | 6,1 | 29 |
| RN-1-192 | 37 | 4,3 | 111 |
| RN-1-196 | 38 | 4,6 | 60 |
| RN-1-197 | 39 | 4,0 | 36 |
| RN-2-001 | 40 | - | 27 |
| RN-2-005 | 41 | 6,7 | 173 |
| RN-2-015 | 42 | 8,1 | 85 |
| RN-2-016 | 43 | 4,6 | 40 |
| RN-2-019 | 44 | 3,6 | 71 |
| RN-2-034 | 47 | 4,1 | 49 |
| RN-3-066 | 54 | - | 27 |
| RN-3-067 | 55 | - | 25 |
| RN-3-068 | 56 | - | 26 |
| RN-3-069 | 57 | - | 25 |
| RN-3-070 | 58 | - | 25 |
| RN-3-089 | 59 | - | 27 |
| RN-3-098 | 60 | - | 320 |
| RN-3-122 | 61 | - | 40 |

Ces essais démontrent que les composés selon l'invention testés présentent une meilleure protection contre la ricine et/ou Stx que Retro-2 cyclisé.

### III. Mise en évidence du blocage du transport rétrograde

### Protocole expérimental

Le transport rétrograde de la sous-unité B de la toxine de Shiga (StxB) à l'appareil de Golgi a été quantifié par un test de sulfatation décrit en détail dans la littérature (Amessou M. *et al. Curr. Protoc. Cell. Biol.* **2006,** *Chapter 15*: Unit 15.10). Le principe est le suivant : un variant de StxB appelé StxB-Sulf₂, qui porte un tandem de sites de reconnaissance de sulfatation protéique, est internalisé en présence de ³⁵SO₄²⁻. Une fois que StxB-Sulf₂ atteint l'appareil de Golgi, les sulfo-transferases localisées dans l'appareil de Golgi catalysent le transfert du sulfate radioactif sur StxB-Sulf₂. Après lyse cellulaire, immuno-précipitation et électrophorèse sur gel, le [³⁵S]-StxB-Sulf₂ peut être détecté et quantifié par autoradiographie. Dans une publication précédente (Stechmann B. et al. Cell 2010, 141, 231-24), il a été montré que le transport rétrograde des cellules traitées avec Rétro-1 ou Retro-2 était diminué de 90% dans certaines conditions expérimentales.

### Résultats

Le Tableau V indique pour les cellules traitées ou non avec différentes molécules, la mesure du transport rétrograde en pourcentage. 100% indique aucun effet sur le transport rétrograde, 0% indique un blocage complet de ce transport.

**Tableau V. Evaluation du transport rétrograde des cellules en présence de composés selon l'invention.**

| **molécule** | **Composé** | **% contrôle** |
|---|---|---|
| DMSO | | 100 |
| Retro 2 | | 36,9 |
| RN-1-068 | 7 | 13,5 |
| RN-1-148 | 13 | 13,1 |
| RN-1-173 | 17 | 22,8 |
| RN-1-174 | 18 | 17,1 |
| RN-1-175 | 19 | 10,7 |
| RN-1-176 | 20 | 6,5 |
| RN-1-177 | 21 | 0,0 |
| RN-1-186 | 36 | 7,1 |
| RN-1-192 | 37 | 10,3 |
| RN-1-196 | 38 | 18,2 |
| RN-1-197 | 39 | 6,2 |
| RN-2-001 | 40 | 5,3 |
| RN-2-005 | 41 | 0,0 |
| RN-2-015 | 42 | 0,0 |

### CONCLUSION

Les composés ci-dessus montrent une activité marquée de blocage du transport rétrograde, supérieure à celle de Retro-2, qui peut s'expliquer soit par une bonne affinité pour la cible et/ou une bonne internalisation dans la cellule soit par une bonne stabilité de ces composés en milieu biologique.

## Revendications

1. Composé de formule générale (I) : 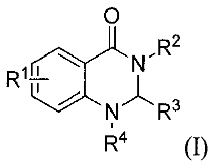 où
**R¹** représente un atome d'hydrogène, un atome d'halogène ou un radical alcoxy de 1 à 3 atomes de carbone ;
**R²** représente :
- un radical phényle, éventuellement substitué par : un radical phényle, un groupe -CF₃, un atome d'halogène, un groupe -SO₂-phényle ou un groupe -S-X ou
- O-X, X étant un radical alkyle de 1 à 4 atomes de carbone, de préférence, X est un radical méthyle ;
- un hétérocycle ou un hétérobicycle de 5 à 10 atomes comprenant un ou deux atomes d'azote, de préférence, un radical pyridine ;
- un cycle adamantyl, éventuellement substitué par une fonction hydroxyle ;
**R³** représente :
- un hétérocycle aromatique de 5 à 6 atomes qui peut être choisi parmi un cycle furane, thiophène, pyrrole, pyrroline, pyrrolidine, dioxolane, oxazole, thiazole, imidazole, imidazoline, imidazolidine, pyrazole, isoxazole, isothiazole, pyrane, pyridine, pipéridine, dioxane, morpholine, pyridazine, pyrimidine, pyrazine, de préférence un cycle thiophène ou pyridine ;
ledit hétérocycle est substitué par :
* un radical alkyle de 1 à 3 atomes de carbone, préférentiellement méthyle ;
* un atome d'halogène ;
* un radical phényle éventuellement substitué par un radical alcoxy de 1 à 3 atomes de carbone, un groupe -CN, un groupe -NO₂ ou un groupe - COX ou -COOX avec X un radical alkyle de 1 à 4 atomes de carbone, ou une combinaison de ces substituants, de préférence, un radical méthyle ;
* un groupe -SY, Y étant un radical alkyle de 1 à 4 atomes de carbone ou un radical phényle ;
* un groupe -CN ;
* un hétérocycle aromatique de 5 ou 6 atomes qui peut être choisi parmi un cycle furane, thiophène, pyrrole, pyrroline, pyrrolidine, dioxolane, oxazole, thiazole, imidazole, imidazoline, imidazolidine, pyrazole, isoxazole, isothiazole, pyrane, pyridine, pipéridine, dioxane, morpholine, pyridazine, pyrimidine, pyrazine, de préférence, ledit hétérocycle de 5 ou 6 atomes est un thiophène, une pyridine, un furane, un thiazole ; éventuellement substitué par au moins un radical alkyle de 1 à 3 atomes de carbone ;
- un radical phényle ; éventuellement substitué par un radical alcoxy de 1 à 3 atomes de carbone ou un groupe NMe₂ ;
**R⁴** représente un atome d'hydrogène ou un radical méthyle ;
à l'exception du composé tel que R¹ est un atome d'hydrogène, R² un radical phényle, R³ un radical 5-méthylthiophèn-2-yl et R⁴ un atome d'hydrogène.

2. Composé de formule générale (I) selon la revendication 1, **caractérisé en ce qu'**il est choisi parmi :
| molécule | composé | Nom |
|---|---|---|
| RN-1-013 | 1 | 2-(3-Methoxyphenyl)-3-phenyl-2,3-dihydroquinazolin-4(1*H*)-one |
| RN-1-019 | 2 | 2-(4-(Dimethylamino)phenyl)-3-phenyl-2,3-dihydroquinazolin-4(1*H*)-one |
| RN-1-021 | 3 | 2,3-Diphenyl-2,3-dihydroquinazolin-4(1*H*)-one |
| RN-1-027 | 4 | 2-(4-Methoxyphenyl)-3-phenyl-2,3-dihydroqunazolin-4(1*H*)-one |
| RN-1-066 | 5 | 2-(3-Methylthiophen-2-yl)-3-phenyl-2,3-dihydroquinazolin-4(1*H*)-one |
| RN-1-067 | 6 | 2-(4-Methylthiophen-2-yl)-3-phenyl-2,3-dihydroquinazolin-4(1*H*)-one |
| RN-1-068 | 7 | 2-(5-Ethylthiophen-2-yl)-3-phenyl-2,3-dihydroquinazolin-4(1*H*)-one |
| RN-1-086 | 8 | 3-((5R,7S)-3-Hydroxyadamantan-1-yl)-2-(5-methylthiophen-2-yl)-2,3-dihydroquinazolin-4(1*H*)-one |
| RN-1-100 | 9 | 2-(5-Bromothiophen-2-yl)-3-phenyl-2,3-dihydroquinazolin-4(1*H*)-one |
| RN-1-118 | 10 | 2-(5-Methylthiophen-2-yl)-3-(pyridin-3-yl)-2,3-dihydroquinazolin-4(1*H*)-one |
| RN-1-120 | 11 | 2-(5-Methylthiophen-2-yl)-3-(pyridin-4-yl)-2,3-dihydroquinazolin-4(1*H*)-one |
| RN-1-132 | 12 | 2-(5-Methylthiophen-2-yl)-3-(pyrimidin-4-yl)-2,3-dihydroquinazolin-4(1*H*)-one |
| RN-1-148 | 13 | 3-Phenyl-2-(5-phenylthiophen-2-yl)-2,3-dihydroquinazolin-4(1*H*)-one |
| RN-1-149 | 14 | 3-([1,1'-Biphenyl]-4-yl)-2-(5-methylthiophen-2-yl)-2,3- |
| | | dihydroquinazolin-4(1*H*)-one |
| RN-1-151 | 15 | 2-(5-Methylthiophen-2-yl)-3-(3-(trifluoromethyl)phenyl)-2,3-dihydroquinazolin-4(1*H*)-one |
| RN-1-162 | 16 | 8-Methyl-2-(5-methylthiophen-2-yl)-3-phenyl-2,3-dihydroquinazolin-4(1*H*)-one |
| RN-1-173 | 17 | 2-(5-(Methylthio)thiophen-2-yl)-3-phenyl-2,3-dihydroquinazolin-4(1*H*)-one |
| RN-1-174 | 18 | 2-([2,2'-Bithiophen]-5-yl)-3-phenyl-2,3-dihydroquinazolin-4(1*H*)-one |
| RN-1-175 | 19 | 3-Phenyl-2-(5-(pyridin-2-yl)thiophen-2-yl)-2,3-dihydroquinazolin-4(1*H*)-one |
| RN-1-176 | 20 | 2-(5-(Furan-2-yl)thiophen-2-yl)-3-phenyl-2,3-dihydroquinazolin-4(1*H*)-one |
| RN-1-177 | 21 | 2-(5-(2-Methylthiazol-4-yl)thiophen-2-yl)-3-phenyl-2,3-dihydroquinazolin-4(1*H*)-one |
| RN-2-120 | 22 | 3-(4-Fluorophenyl)-2-(5-methylthiophen-2-yl)-2,3-dihydroquinazolin-4(1*H*)-one |
| RN-2-137 | 23 | 3-(2-Fluorophenyl)-2-(5-methylthiophen-2-yl)-2,3-dihydroquinazolin-4(1*H*)-one |
| RN-2-161 | 24 | 6-Methoxy-2-(5-methylthiophen-2-yl)-3-phenyl-2,3-dihydroquinazolin-4(1*H*)-one |
| RN-2-162 | 25 | 6-Fluoro-2-(5-methylthiophen-2-yl)-3-phenyl-2,3-dihydroquinazolin-4(1*H*)-one |
| RN-2-163 | 26 | 7-Fluoro-2-(5-methylthiophen-2-yl)-3-phenyl-2,3-dihydroquinazolin-4(1*H*)-one |
| RN-2-177 | 27 | 3-(4-Bromophenyl)-2-(5-methylthiophen-2-yl)-2,3-dihydroquinazolin-4(1*H*)-one |
| RN-2-178 | 28 | 3-(3-Bromophenyl)-2-(5-methylthiophen-2-yl)-2,3-dihydroquinazolin-4(1*H*)-one |
| RN-2-180 | 29 | 3-(4-Chlorophenyl)-2-(5-methylthiophen-2-yl)-2,3-dihydroquinazolin-4(1*H*)-one |
| RN-2-181 | 30 | 3-(3-Chlorophenyl)-2-(5-methylthiophen-2-yl)-2,3-dihydroquinazolin-4(1*H*)-one |
| RN-2-183 | 31 | 3-(2-(Methylthio)phenyl)-2-(5-methylthiophen-2-yl)-2,3-dihydroquinazolin-4(1*H*)-one |
| RN-2-182 | 32 | 3-(2-Chlorophenyl)-2-(5-methylthiophen-2-yl)-2,3-dihydroquinazolin-4(1*H*)-one |
| RN-2-199 | 33 | 6-Iodo-2-(5-methylthiophen-2-yl)-3-phenyl-2,3-dihydroquinazolin-4(1*H*)-one |
| RN-3-121 | 34 | 6-fluoro-2-(5-(2-methylthiazol-4-yl)thiophen-2-yl)-3-phenyl-2,3-dihydroquinazolin-4(1H)-one |
| RN-1-181 | 35 | 1-Methyl-2-(5-methylthiophen-2-yl)-3-phenyl-2,3-dihydroquinazolin-4(1*H*)-one |
| RN-1-186 | 36 | 1-Methyl-3-phenyl-2-(5-phenylthiophen-2-yl)-2,3-dihydroquinazolin-4(1*H*)-one |
| RN-1-192 | 37 | 1-Methyl-3-phenyl-2-(5-(pyridin-2-yl)thiophen-2-yl)-2,3-dihydroquinazolin-4(1*H*)-one |
| RN-1-196 | 38 | 1-Methyl-2-(5-(methylthio)thiophen-2-yl)-3-phenyl-2,3-dihydroquinazolin-4(1*H*)-one |
| RN-1-197 | 39 | 2-([2,2'-Bithiophen]-5-yl)-1-methyl-3-phenyl-2,3-dihydroquinazolin-4(1*H*)-one |
| RN-2-001 | 40 | 2-(5-(Furan-2-yl)thiophen-2-yl)-1-methyl-3-phenyl-2,3-dihydroquinazolin-4(1*H*)-one |
| RN-2-005 | 41 | 2-(5-Ethylthiophen-2-yl)-1-methyl-3-phenyl-2,3 -dihydroquinazolin-4(1*H*)-one |
| RN-2-015 | 42 | 1-Methyl-2-(5-(2-methylthiazol-4-yl)thiophen-2-yl)-3-phenyl-2,3-dihydroquinazolin-4(1*H*)-one |
| RN-2-016 | 43 | 2-(5-Bromothiophen-2-yl)-1-methyl-3-phenyl-2,3-dihydroquinazolin-4(1*H*)-one |
| RN-2-019 | 44 | 1-Methyl-3-phenyl-2-(5-(phenylthio)thiophen-2-yl)-2,3-dihydroquinazolin-4(1*H*)-one |
| RN-2-029 | 45 | 2-(5-(3,4-Dimethoxyphenyl)thiophen-2-yl)-1-methyl-3-phenyl-2,3-dihydroquinazolin-4(1*H*)-one |
| RN-2-032 | 46 | 1-Methyl-2-(5-(3-nitrophenyl)thiophen-2-yl)-3-phenyl-2,3-dihydroquinazolin-4(1*H*)-one |
| RN-2-034 | 47 | 2-(5-(Furan-3 -yl)thiophen-2-yl)-1-methyl-3 -phenyl-2,3-dihydroquinazolin-4(1*H*)-one |
| RN-2-049 | 48 | Methyl 4-(5-(1-methyl-4-oxo-3-phenyl-1,2,3,4-tetrahydroquinazolin-2-yl)thiophen-2-yl)benzoate |
| RN-2-050 | 49 | 2-(5-(4-Acetylphenyl)thiophen-2-yl)-1-methyl-3-phenyl-2,3-dihydroquinazolin-4(1*H*)-one |
| RN-2-053 | 50 | 1-Methyl-3-phenyl-2-(5-(3,4,5-trimethoxyphenyl)thiophen-2-yl)-2,3-dihydroquinazolin-4(1*H*)-one |
| RN-2-057 | 51 | 1-methyl-2-(5-(4-methyl-3-nitrophenyl)thiophen-2-yl)-3-phenyl-2,3-dihydroquinazolin-4(1H)-one |
| RN-2-059 | 52 | 4-(5-(1-Methyl-4-oxo-3-phenyl-1,2,3,4-tetrahydroquinazolin-2-yl)thiophen-2-yl)benzonitrile |
| RN-3-012 | 53 | 5-(1-Methyl-4-oxo-3-phenyl-1,2,3,4-tetrahydroquinazolin-2-yl)thiophene-2-carbonitrile |
| RN-3-066 | 54 | 3-(2-chlorophenyl)-1-methyl-2-(5-methylthiophen-2-yl)-2,3-dihydroquinazolin-4(1H)-one |
| RN-3-067 | 55 | 3-(2-iodophenyl)-1-methyl-2-(5-methylthiophen-2-yl)-2,3-dihydroquinazolin-4(1H)-one |
| RN-3-068 | 56 | 3-(2-methoxyphenyl)-1-methyl-2-(5-methylthiophen-2-yl)-2,3-dihydroquinazolin-4(1H)-one |
| RN-3-069 | 57 | 1-methyl-3-(2-(methylthio)phenyl)-2-(5-methylthiophen-2-yl)-2,3-dihydroquinazolin-4(1H)-one |
| RN-3-070 | 58 | 3-(2-fluorophenyl)-1-methyl-2-(5-methylthiophen-2-yl)-2,3-dihydroquinazolin-4(1H)-one |
| RN-3-089 | 59 | 3-([1,1'-biphenyl]-2-yl)-1-methyl-2-(5-methylthiophen-2-yl)-2,3-dihydroquinazolin-4(1H)-one |
| RN-3-098 | 60 | 1-methyl-2-(5-methylthiophen-2-yl)-3-(2-(phenylsulfonyl)phenyl)-2,3-dihydroquinazolin-4(1H)-one |
| RN-3-122 | 61 | 6-fluoro-1-methyl-2-(5-(2-methylthiazol-4-yl)thiophen-2-yl)-3-phenyl-2,3-dihydroquinazolin-4(1H)-one |

3. Composé de formule générale (I) selon la revendication 1, **caractérisé en ce que** :
- R¹ est un atome d'hydrogène ou un atome de fluor ;
- R² est un cycle phényle éventuellement substitué par : un atome de chlore, d'iode ou de fluor, un radical -OCH₃, -SCH₃, -SO₂-phényle ou un phényle ;
- R³ est un radical thiophène substitué par un radical méthyle, éthyle, phényle, -SCH₃, 2-thiophène, 2-furane, 3-furane, 2-pyridine, 4-(2-Me thiazole), S-phényle ou un atome de brome, et
- R⁴ est un atome d'hydrogène ou un radical méthyle ;
à l'exception du composé tel que R¹ est un atome d'hydrogène, R² un radical phényle, R³ un radical 5-méthyl thiophèn-2-yl et R⁴ un atome d'hydrogène.

4. Composé de formule générale (I) selon l'une quelconque des revendications précédentes, **caractérisé en ce qu'**il est choisi parmi les composés 7, 9, 13, 17, 18, 19, 20, 21, 25, 31, 32, 34, 35, 36, 37, 38, 39, 40, 41, 42, 43, 44, 47, 54, 55, 56, 57, 58, 59, 60, 61.

5. Composé de formule générale (I) selon l'une quelconque des revendications précédente pour son utilisation pour la prévention et/ou le traitement des désordres induits par les toxines à mode d'action intracellulaire utilisant le transport rétrograde.

6. Composé de formule générale (I) selon l'une quelconque des revendications 1 à 4 pour son utilisation selon la revendication 5, **caractérisé en ce que** lesdites toxines à mode d'action intracellulaire sont choisies parmi la ricine, la toxine de Shiga et les toxines Shiga-like (Stxs) produite par *Shigella dysenteriae* (Stx) et *E. coli* (Stx1 et Stx2), la toxine cholérique (Ctx de *Vibrio cholerae* responsable du choléra), la toxine pertussique *(Bordetella pertussis* agent de la coqueluche), la cytotoxine subtilase et l'entérotoxine thermolabile *(E. coli*).

7. Composition pharmaceutique ou médicament comprenant au moins un composé de formule générale (I) tels que définis dans l'une quelconque des revendications 1 à 4, dans un véhicule pharmaceutiquement acceptable, **caractérisé en ce que** ladite composition pharmaceutique ou ledit médicament est adapté à une administration par les voies aérienne, orale, parentérale ou locale.

8. Composition pharmaceutique ou médicament comprenant au moins un composé de formule générale (I) tels que définis dans l'une quelconque des revendications 1 à 4 pour une utilisation selon la revendication 5 ou la revendication 6, dans un véhicule pharmaceutiquement acceptable, **caractérisé en ce que** ladite composition pharmaceutique ou ledit médicament est adapté à une administration par les voies aérienne, orale, parentérale ou locale.
